Europäisches Patentamt

European Patent Office

Office européen des brevets

(11) Numéro de publication: **0 253 742 B1**

(12) **FASCICULE DE BREVET EUROPEEN**

(45) Date de publication de fascicule du brevet: **14.10.92**  (51) Int. Cl.⁵: **A61B 6/00**

(21) Numéro de dépôt: **87401674.4**

(22) Date de dépôt: **16.07.87**

(54) **Appareil de détermination du contenu minéral osseux.**

(30) Priorité: **18.07.86 FR 8610492**

(43) Date de publication de la demande:
**20.01.88 Bulletin 88/03**

(45) Mention de la délivrance du brevet:
**14.10.92 Bulletin 92/42**

(84) Etats contractants désignés:
**AT BE CH DE ES GB GR IT LI LU NL SE**

(56) Documents cités:
**EP-A- 0 077 527**

**JOURNAL OF NUCLEAR MEDICINE, vol. 25, no. 11, novembre 1984, pages 1241-1253, Mayo Foundation, New York, US; H.W. WAHNER et al.: "Assessment of bone mineral. Part 2"**

**MEDICAL PHYSICS, vol. 9, no. 2, mars-avril 1982, pages 208-215, Am. Assoc. Phys. Med., New York, US; S.-S. LING et al.: "The measurement of trabecular bone mineral density using coherent and Compton scattered photons in vitro"**

(73) Titulaire: **COMMISSARIAT A L'ENERGIE ATOMIQUE Etablissement de Caractère Scientifique Technique et Industriel
31/33, rue de la Fédération
F-75015 Paris(FR)**

(72) Inventeur: **Tanguy, Jean-Claude
17, Avenue du Château Chaiges
F-91200 Athis Mons(FR)**
Inventeur: **Chambellan, Dominique
39, Avenue des Etats-Unis
F-78000 Versailles(FR)**
Inventeur: **Pommet, Raymond
12, rue du Colombier
F-78690 les Essarts le Roi(FR)**

(74) Mandataire: **Mongrédien, André et al
c/o BREVATOME 25, rue de Ponthieu
F-75008 Paris(FR)**

# Description

La présente invention a pour objet un appareil de détection et de visualisation du contenu minéral de tissus osseux, appelé ostéodensimètre en langage spécialisé, ainsi que, selon une utilisation plus particulière, l'application de cet appareil à un procédé efficient et précis de mesure de la déminéralisation de la colonne vertébrale.

Il est en effet connu depuis longtemps que la minéralisation du squelette humain augmente parallèlement à la croissance de la taille de l'individu, et peut se prolonger encore un peu après l'arrêt de cette croissance, jusqu'à ce que les os atteignent un poids maximal vers la trentaine. Après cette période, le vieillissement est témoin du phénomène inverse : les os ne se renouvellent pas complètement et leur poids diminue progressivement. Les causes de ce processus ne sont pas complètement élucidées et sont de toute façon nombreuses, la constitution génétique du sujet et la richesse de son régime en calcium jouant par exemple un rôle important. De plus ce phénomène de déminéralisation, outre qu'il touche très irrégulièrement les individus, est sélectif d'après les os : la colonne vertébrale, le radius et le fémur y sont très sensibles alors que d'autres os restent intacts avec l'âge ; les femmes y sont en principe beaucoup plus sujettes que les hommes ; enfin, la déminéralisation peut être linéaire en fonction du temps ou de plus en plus rapide. Le degré pathologique de ce processus constitue l'ostéoporose.

L'os atteint devient petit à petit poreux et finit par perdre ses caractéristiques mécaniques. Il peut devenir inapte à assumer son rôle, et c'est alors l'affaissement du tronc du malade joint à des douleurs vertébrales, la déformation rachidienne ou même la rupture du col du fémur. Cette dernière maladie entraîne une mortalité de 25% et une invalidité importante dans le cas contraire. Les coûts pour la santé publique sont donc très élevés et ne peuvent que s'alourdir par suite du vieillissement de la population dans les pays développés. La prévention peut être très avantageuse, ce qui requiert toutefois des moyens de détection adaptés à l'importance de la population à isque (30% des femmes ménopausées sont atteintes d'ostéoporose).

Les procédés de diagnostic connus impliquent tous l'irradiation des parties du corps exposées. La technique, courante pour d'autres applications, des rayons X est toutefois insuffisante pour un diagnostic précis et ne peut fournir que des indications générales dans des cas de déminéralisation déjà avancée : les mesures sont affectées par suite de la difficulté à étalonner fidèlement la source de rayonnement et les caractéristiques d'exposition et de traitement du film.

On a ainsi développé les techniques de mesures par rayonnement gamma qui présentent le double avantage d'être peu irradiantes pour les tissus exposés, et de pouvoir être recueillis par des capteurs tels que des détecteurs à scintillation aptes à traduire l'absorption partielle du rayonnement. Des sources de gadolinium 153 ont plus particulièrement été employées en effet ce corps présente l'avantage d'émettre un rayonnement suivant deux énergies différentes, qui se comportent différemment vis-à-vis des os et des tissus mous et permettent donc de distinguer l'énergie absorbée par ces tissus de celle absorbée en fonction de l'épaisseur de l'os, qui seule intéresse le médecin.

Cette distinction est effectuée de façon satisfaisante grâce à un analyseur intégré dans la chaîne de mesure, et il n'est donc plus nécessaire d'utiliser les artifices, nécessaires avec des sources à rayonnement à une seule énergie, tels que plonger la partie du corps à examiner dans un récipient d'eau pour simuler une épaisseur constante de tissus mous, ce qui est valable en soi mais peu pratique si l'on s'intéresse à une colonne vertébrale entière.

On peut cependant envisager de remplacer le gadolinium 153 car les rayonnements qu'il émet sont assez sensibles à la graisse irrégulièrement contenue dans les tissus traversés, et on est donc amené à utiliser une source double. Les auteurs de l'invention ont examiné ce problème auparavant et préconisent l'emploi d'américium 241 (énergie 60 keV, demi-vie 432 ans) et de baryum 133 (356 keV, 10,6 ans) comme posant peu de problèmes de protection radiologique et très peu sensible à la présence de graisse dans les tissus (d'après "Mesures de la masse minérale osseuse par atténuation de photons gamma dichromatiques", par D. Tola, R. Hours et J. Boutaine, dans "Mesure et signification du volume osseux et de la masse osseuse", par Laval-Jeantet et Caulin, publié par Armour-Montagu en 1982). Cette technique ainsi améliorée donne des résultats d'une précision suffisante, alors que la reconstitution de l'image de l'organisme en profondeur par tomographie, qui constitue le meilleur procédé d'investigation actuel, nécessite un appareillage coûteux complété par un ordinateur et reste une technique trop sophistiquée pour une simple mesure d'ostéoporose.

Des problèmes supplémentaires, qui sont à l'origine de la présente invention, se posent cependant dans le cas de l'application, très importante, de la technique à l'examen de la colonne vertébrale. Il est en effet nécessaire d'allonger le patient en décubitus dorsal, les jambes légèrement surélevées, de façon à diminuer la lordose lombaire ; dans ces conditions, la disposition la plus commode et la moins encombrante consiste à placer la source radioactive au-dessus du patient et le bloc récepteur au-dessous ou vice-versa ; les rayons émis sont donc verticaux et on obtient des images de face qui présentent le désavantage de superposer l'image du corps vertébral, qui intéresse principalement le médecin, à celle de l'arc postérieur qui est moins sujet à la déminéralisation. La précision des relevés est donc affectée par la présence de cet arc postérieur, d'autant plus que sa forme est très irrégulière.

On pourrait alors être tenté de disposer le bloc émetteur, porteur de la source radioactive, et le bloc récepteur sur les côtés du patient de façon à obtenir une image de profil qui isolerait le corps vertébral, mais une autre difficulté apparaît alors à cause de la difficulté de recueillir de façon à satisfaisante un rayonnement sur une bande continue de détecteurs à scintillation par suite de l'encombrement des détecteurs utilisés jusqu'ici. L'image est donc construite point par point à l'aide d'un seul capteur situé en regard de la source, ce qui oblige, outre à des immobilisations prolongées, à prévoir un double mouvement de translation de la source et du capteur qui est beaucoup plus facile à accomplir dans le cas d'un faisceau vertical, car les translations se font alors dans le plan horizontal et seuls des moyens mécaniqus simples sont requis, ce qui ne serait pas le cas s'il était nécessaire de prévoir un mouvement de va-et-vient vertical d'un lourd ensemble.

Le document EP-A-0 077 527 décrit un appareil de radiologie générale qui, grâce à un bras articulé portant la source de rayons X et l'un des deux détecteurs de l'appareil, permet de prendre deux clichés orthogonaux.

La présente invention a pour but de remédier aux divers inconvénients cités à propos de l'examen de la colonne vertébrale ; elle a pour objet un appareil de détermination du contenu minéral osseux par absorption partielle de rayonnements ionisants, comprenant un bloc constitué d'une source radioactive et un autre bloc constitué d'un détecteur de rayonnement placé vis-à-vis de la source en position de travail et séparé de celle-ci d'une distance suffisante pour permettre l'introduction de la matière organique à examiner, caractérisé en ce qu'un des blocs est lié par une articulation à un premier bras, lui-même articulé sur un chariot mobile de l'appareil, tandis que l'autre bloc est solidaire d'un second bras articulé lui aussi sur le chariot mobile.

L'axe de l'articulation du premier de ces bras est de préférence parallèle à la direction de déplacement du chariot tandis que l'axe de l'articulation du second bras est perpendiculaire à cette direction et fait un angle de 45° avec la verticale.

Il est donc possible de disposer l'appareil de façon à orienter le faisceau horizontalement ou verticalement, alors qu'une seule translation du chariot est requise car, suivant un mode de réalisation préferée de l'invention, le bloc récepteur comprend une pluralité de capteurs disposés en une ou plusieurs rangées perpendiculaires à la direction de déplacement du chariot mobile, si bien que leur translation suivant cette direction permet l'obtention d'images à deux dimensions.

La source radioactive est à deux énergies de façon à distinguer l'absorption due aux os de celle due aux tissus mous ; on préconise l'emploi d'un rayonnement soit par deux éléments radioactifs de demi-vie longue, tels que l'américium 241 et le baryum 133 (ou un seul, tel que le césium 137 couplé à un rayonnement X) ou encore le gadolinium 153 déjà répandu auparavant.

L'appareil ainsi conçu est plus particulièrement applicable à un nouveau procédé de détermination du contenu osseux de la colonne vertébrale, caractérisé en ce qu'il consiste à déplacer selon deux opérations de balayage le chariot mobile le long d'un patient en décubitus dorsal sur une table d'examen, les blocs étant disposés au-dessus et au-dessous de lui pour un balayage, à sa gauche et à sa droite pour l'autre balayage, la table étant retirée entre ces deux opérations pour permettre la rotation des deux bras ; il consiste finalement à corriger par des moyens numériques les intervalles de temps entre la prise d'informations concernant les différents points situés à la même hauteur du patient par les différents capteurs du bloc détecteur.

Une réalisation possible de l'invention, adaptée plus spécialement au procédé que l'on vient de décrire, est commentée concrètement ci-dessous à l'aide des figures suivantes, données à titre illustratif et non limitatif :

- la figure 1 représente un schéma d'installation par absorptiomètrie d'après l'art antérieur ;
- la figure 2 représente une vue en perspective de l'invention illustrant notamment sa cinématique ;
- la figure 3 représente une vue de dessus d'un des bras articulés de l'invention, dont la figure 4 donne la forme générale avec une légère perspective ;
- la figure 5 représente le système de maintien en place de ce bras ;
- la figure 6 représente le système de maintien en place et de pivotement de l'autre bras ;
- la figure 7 représente une vue du bloc émetteur avec coupe partielle ;
- les figures 8 et 9 représentent des vues de côté et de face avec coupes partielles du dispositif d'entraînement et de guidage du chariot ;
- la figure 10 représente le bloc récepteur et en particulier la position des capteurs ; et

- la figure 11 représente le bloc récepteur et en particulier la position des capteurs dans une autre réalisation de l'invention.

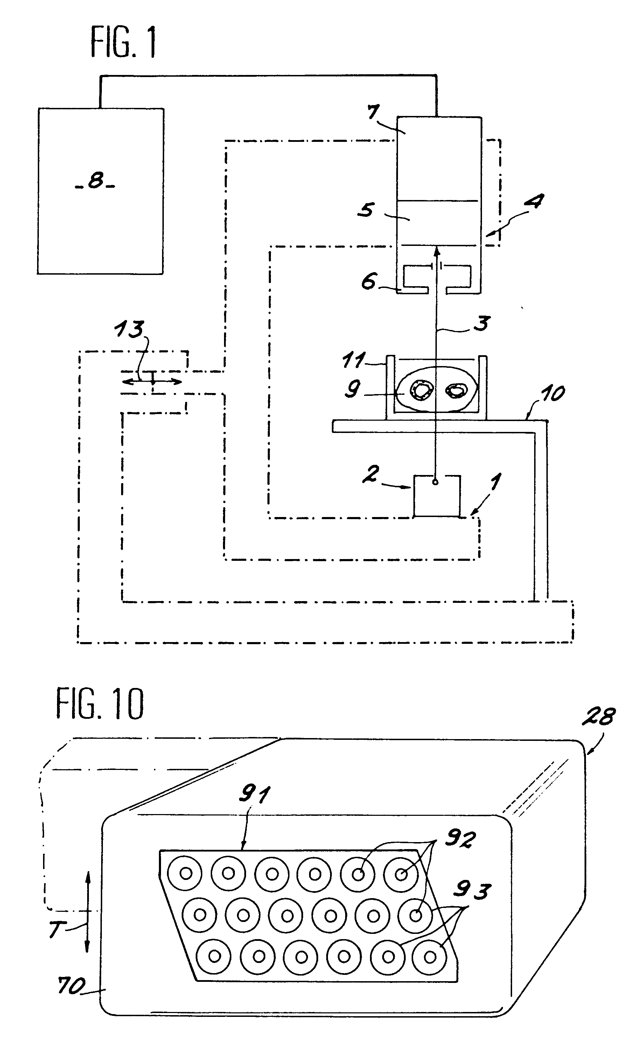

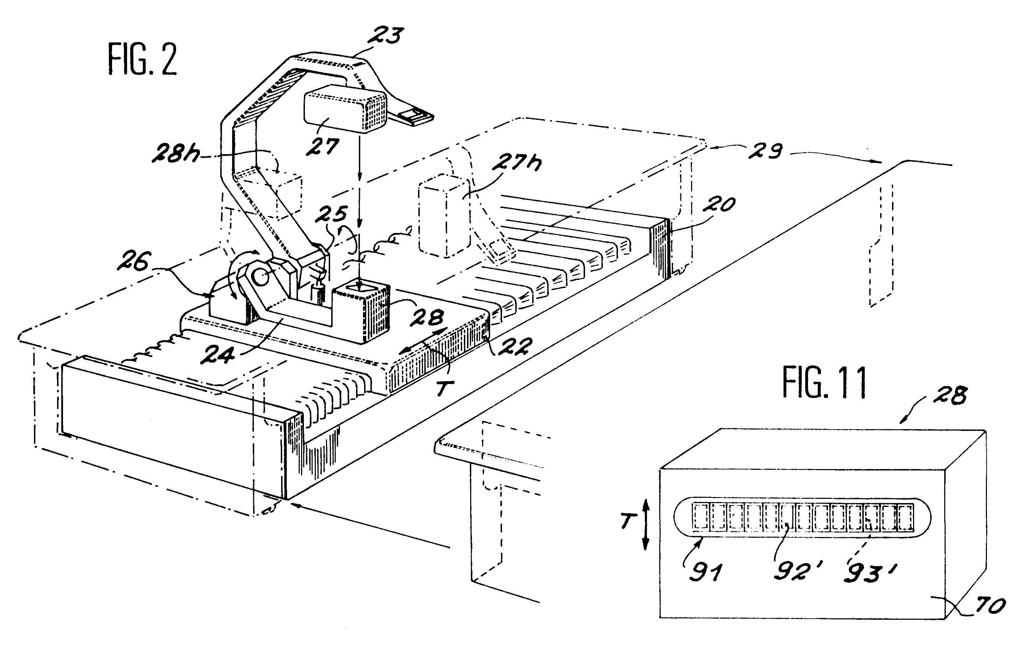

La figure 1 rappelle donc le principe d'ostéodensimètres par absorptiomètrie construits d'après les conceptions antérieures.

Sur un chariot mobile 1 est disposée une source radioactive 2 émettant un faisceau parallèle 3 vers un récepteur 4 situé lui aussi sur le chariot 1 et constitué d'un scintillateur 5 sensible au rayonnement, protégé par un collimateur 6 et complété par un photomultiplicateur 7 lui-même relié à une chaîne de mesure 8 que l'on ne décrira pas ici en détail mais qui comprend essentiellement un analyseur de lumière et un moyen de visualisation graphique.

Sur le chemin du faisceau 3 se trouve le corps organique à analyser, ici un avant-bras humain 9 que l'on apercoit en coupe ainsi que son radius et son cubitus. Cet avant-bras 9 repose sur la partie fixe 10 de l'appareil ; dans le cas d'un ostéodensimètre à une source radioactive, il est en outre nécessaire de simuler une épaisseur constante de tissus mous traversés par le faisceau ; le procédé le plus simple consiste à plonger l'avant-bras dans un réservoir 11 rempli d'eau, ce qui serait évidemment beaucoup plus encombrant à appliquer à l'examen de la colonne vertébrale, et moins efficace à cause de l'hétérogénéité des tissus traversés.

La mesure consiste donc à déplacer le chariot 1 par rapport au bâti 10 selon le sens des flèches 13. On obtient donc une répartition de l'épaisseur osseuse traversée par le faisceau 3 sur la largeur de l'avant-bras 9, c'est-à-dire une information traduisible par une courbe ou encore une ligne de points colorés en fonction de cette épaisseur traversée, suivant le principe employé pour les radiographies par rayons X. Les images à deux dimensions sont obtenues par juxtapositions de telles lignes par déplacement progressif du chariot 1 dans une direction perpendiculaire au plan de la figure 1, ce qui est un processus lent au cours duquel les déplacements du corps à analyser sont inévitables.

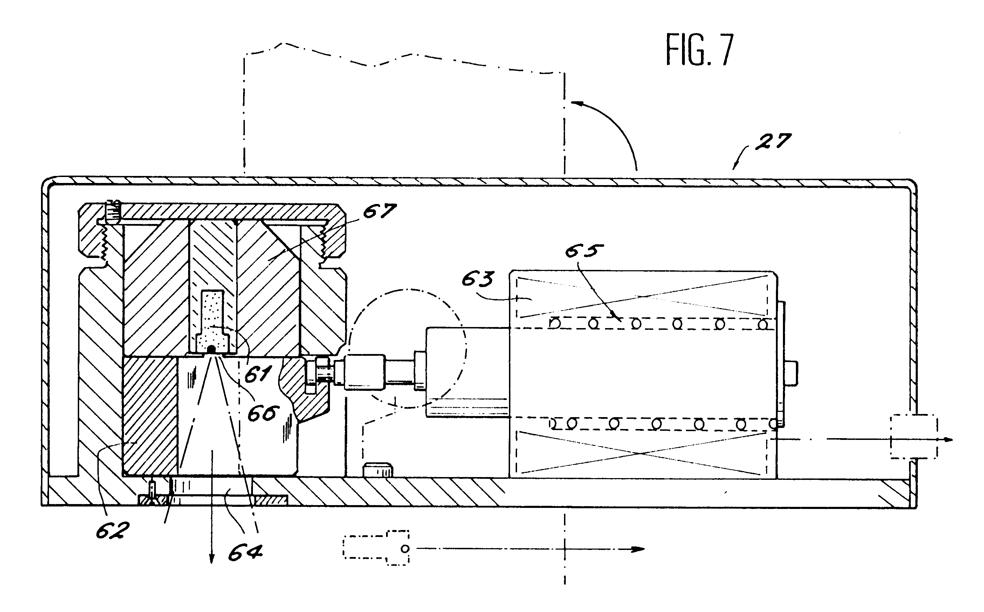

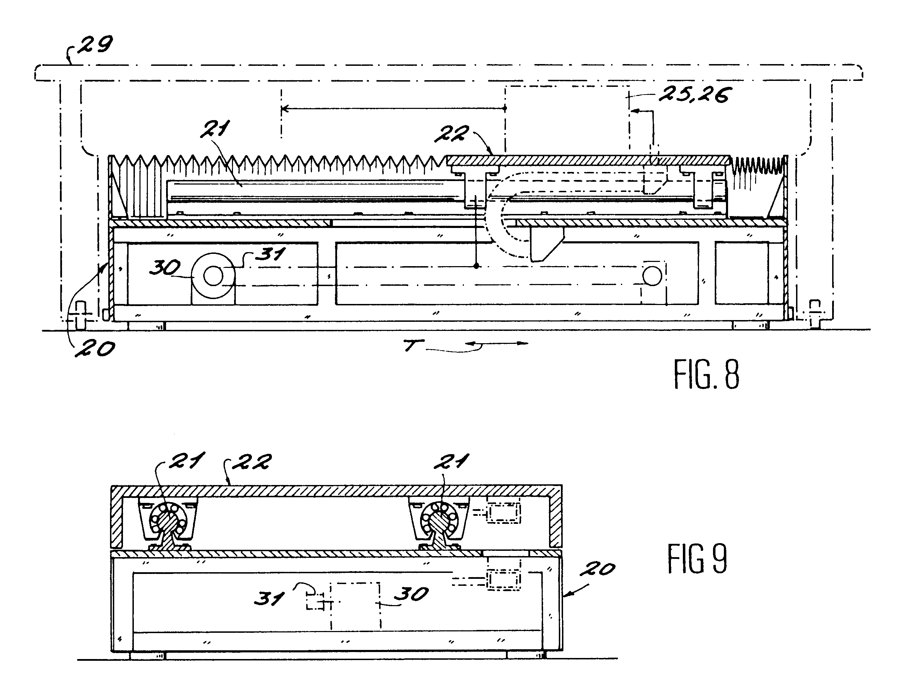

La réalisation de l'invention représentée figure 2 comprend tout d'abord un bâti 20 sur lequel sont fixées des glissières 21 représentées figures 8 et 9 ; elles constituent le support coulissant d'un chariot mobile 22 muni de deux bras 23 et 24 articulés en 25 et 26 sur le chariot 22. Une des articulations 25 possède un axe colinéaire à celui des glissières 21, l'autre articulation 26 appartient au plan perpendiculaire à la précédente et fait un angle de 45° avec la verticale. A l'extrémité du bras 23 est fixé le bloc émetteur 27 porteur de la source de rayonnements ionisants 61 (figure 7) qui peut inclure deux éléments de demi-vie supérieure à cinq ans tels que l'américium 241 et le baryum 133 ( des rayons X peuvent aussi être employés avec du césium 137,ou encore le gadolinium 153); et muni d'une enceinte de protection en métal dense, alors que le bloc récepteur 28 qui contient les capteurs de rayonnement est fixé à l'extrémité de l'autre bras 24. Toutefois, on pourrait envisager l'agencement inverse sans nuire à l'esprit de l'invention.

Deux positions préférentielles peuvent être définies : dans la première, l'axe du faisceau émis est vertical et les blocs sont à la position définie en 27 et 28 sur le dessin. C'est la solution qui a été adoptée par l'art antérieur. Dans la deuxième position préférentielle, l'axe du faisceau émis est horizontal et les blocs émetteur et récepteur sont disposés suivant 27h et 28h. Les opérations de rotation peuvent s'effectuer quand la table 29 sur laquelle est allongé le patient a été déplacée.

Le déplacement du chariot 22 est assuré classiquement par un moteur 30 qui entraîne une courroie 31.

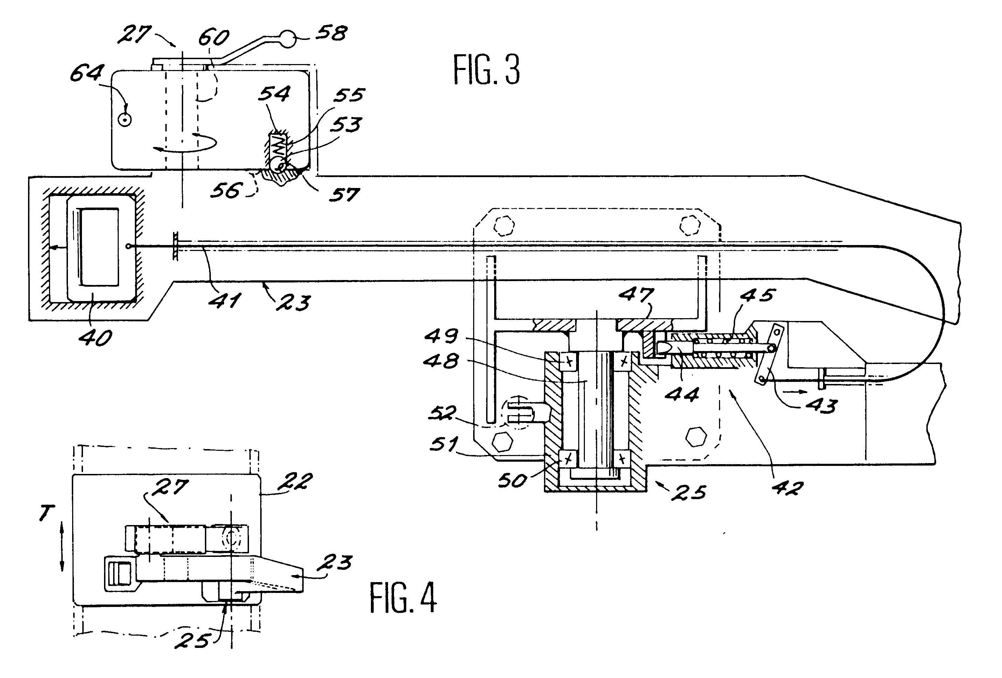

Le bras 23, l'articulation 25 et le bloc émetteur 27 représentés encore schématiquement figure 4 sont décrits plus en détail figures 3, 5 et 7. Le bras 23 est muni d'une poignée 40 dont l'action débloque un verrou 42 par l'intermédiaire d'un câble métallique 41 qui parcourt le bras 23. Ce verrou 42 comprend plus précisément un levier 43 qui réduit la course du câble 41 et entraîne le retrait d'un doigt 44 susceptible d'être rappelé à sa position initiale par un ressort 45 dès que la poignée 40 n'est plus actionnée. Le doigt 44 permet de bloquer le bras 23 dans les deux positions préférentielles de mesure en s'insérant dans une des deux encoches 45 et 46 d'un verrou 47 fixe. L'articulation 25 elle-même se compose d'un axe 48 lié au chariot 22 de l'appareil, de deux roulements à billes 49 et 50 à contact oblique qui permettent la rotation de l'extrémité alésée 51 du bras 23. Le mouvement du bras 23 est facilité par un vérin 52 lié à cette extrémité 51 et assuré par la poignée 40.

Comme les directions préférentielles de mesure sont perpendiculaires et que l'angle de rotation imprimé au bras 23 est inférieur à 90°, il faut tourner le bloc émetteur 27 du complément. Un système simple, tel qu'une bille de butée 53 disposée dans un logement 54 du bloc 27, poussée par un ressort 55 et disposable dans deux enfoncements 56 et 57 de forme complémentaire situés sur le bras 23, peut être employé pour obtenir la stabilisation de l'axe du faisceau ionisant, la rotation du bloc 27 étant assurée par un manche 58 lié à celui-ci et apte à le mouvoir autour d'un axe 60 lié au bras 23.

La source de rayonnements ionisants 61 est disposée à l'intérieur d'un logement de protection biologique 67 percé d'une lumière 66 ; au cours de l'utilisation de l'appareil la source 61 peut émettre un rayonnement par déplacement d'un écran 62 commandé par l'excitation d'un électro-aimant 63. Le collimateur 64 est suffisamment large pour permettre l'établissement d'un faisceau divergent et non rectiligne comme c'était le cas sur la figure 1. L'explication de cette modification sera donnée plus loin.

Un ressort de rappel 65 permet d'aveugler la source ionisante 61 dès que l'électro-aimant 63 n'est plus excité.

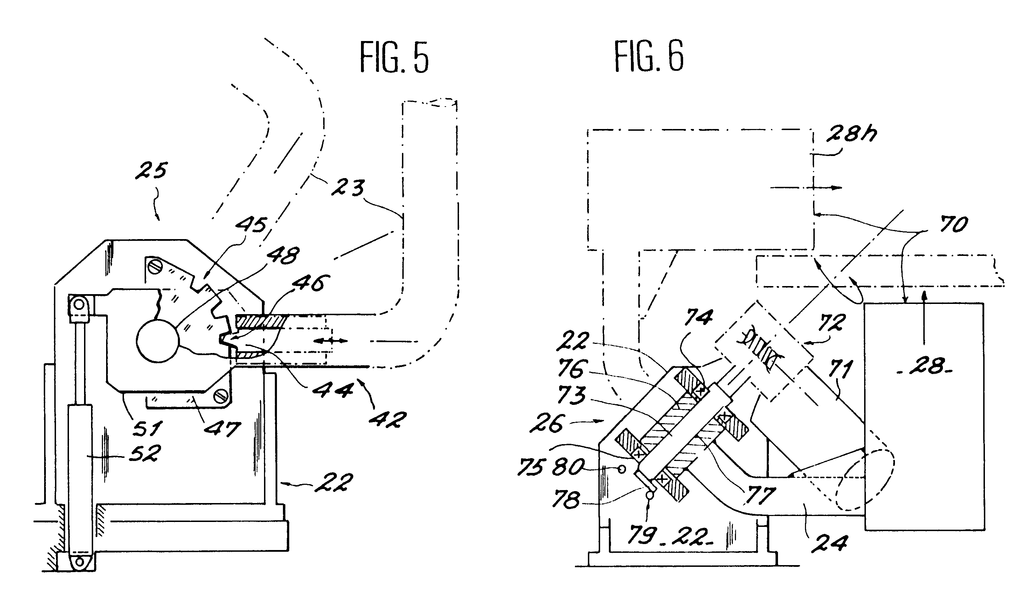

Le mécanisme de rotation du bras 24 et du bloc récepteur 28 est assez différent, comme on le voit sur la figure 6 : le bras 24 effectue un demi-tour pour passer d'une position préférentielle de mesure à l'autre, ce qui implique une rotation de 90° du bloc récepteur 28 et de sa face de mesure 70, si bien qu'il est possible de le fixer rigidement sur ce bras. La rotation est effectuée à l'aide d'un moteur 71 couplé à un motoréducteur 72 ; le mouvement de rotation communiqué à l'arbre 73, soutenu par le chariot 22 au moyen de deux roulements à billes à contact oblique 74 et 75, est repris par l'extrémité alésée 76 du bras 24 au moyen d'un ajustement à jeu négatif 77. Un ergot 78 sur l'arbre 73 effleure tour à tour deux contacts diamétralement opposés 79 et 80 qui permettent d'arrêter le moteur 71 et de stabiliser le bloc récepteur 28 sur l'une ou l'autre des positions souhaitées.

Si l'on se reporte à présent la figure 10, on constate que la face 70 du bloc récepteur 28 présente un écran 91 en forme de parallélogramme derrière lequel sont disposées plusieurs rangées de scintillateurs 92 couplés à autant de photomultiplicateurs 93 situés derrière eux et nettement plus encombrants. Chaque couple définit un capteur de rayonnement.

Les produits actifs des scintillateurs 92 peuvent être les composés NaI, CsI, BiGeO, CsF, CaFr, CdTe et HgI$_2$ en particulier.

Comme d'après cette invention le chariot 22 est animé d'une translation unique selon la double flèche T, et que le patient reste immobile sur la table 29, une image en deux dimensions ne peut être obtenue que par balayage d'une batterie de capteurs qui enregistrent chacun simultanément l'information fournie par une partie du faisceau émis par la source ionisante 61. L'idée la plus immédiate consisterait à disposer les capteurs suivant une ligne tracée sur la face 70 et perpendiculaire à la direction T. Elle n'est guère réalisable par suite de l'encombrement des photomultiplicateurs conventionnels 93 ou de dispositifs équivalents que l'on pourrait employer à leur place, tels que les photodiodes ou les semi-conducteurs : la résolution des images serait trop faible.

Pour résoudre ce problème, on utilise donc des capteurs disposés sur plusieurs rangées, trois sur la figure 10, qui comprennent chacun six scintillateurs 92. Les inventeurs considèrent que d'autres configurations peuvent également conduire à de bons résultats. Cette disposition nécessite évidemment un collimateur 64 de forme adaptée. Elle implique encore que des informations recueillies simultanément ne concernent pas des points situés à la même hauteur du patient. Il faut en tenir compte dans l'organigramme de création de l'image au moyen, par exemple, d'un temporisateur situé sur la chaine de mesures, l'intervalle entre deux rangées perpendiculaires à la direction T étant équivalent à un intervalle de temps par suite de la translation du chariot 22. Bien entendu, il faut aussi prévoir par un étalonnage préliminaire d'éventuelles disparités de l'intensité de rayonnement sur chaque capteur.

D'autres répartitions des capteurs, telles que les quinconces, peuvent être envisagées sans sortir du cadre de l'invention.

Il faut toutefois noter que des photomultiplicateurs 93′ à photocathodes multiples, connus depuis peu, possèdent un encombrement très réduit et permettent de s'affranchir de ce problème de résolution d'image. Avec de tels photomultiplicateurs, on peut se contenter d'une seule rangée de capteurs au nombre, par exemple, de vingt-quatre (figure 11), perpendiculaires au déplacement du chariot 22 pour obtenir une image satisfaisante, les scintillateurs 92′, de forme rectangulaire dans ce cas, se joignant en une rangée continue et recouvrant les photomultiplicateurs 93′. Le procédé de création de l'image est alors simplifié.

On constate donc que l'invention apporte une solution intéressante aux problèmes actuellement posés par une mesure précise de la déminéralisation osseuse, principalement des vertèbres qui sont parmi les os les plus touchés : dans ce cas précis, on peut commencer par vouloir tirer une image de la colonne vertébrale en incidence verticale comme il est habituel : les blocs émetteur 27 et récepteur 28 sont disposés comme sur la figure 2, on effectue la mesure par déplacement du chariot 22, après quoi la table 29 est écartée, le bloc récepteur prend la position indiquée en 28h, la table 29 est remise en place et le bloc émetteur est déplacé jusqu'à la position 27h ; une image de profil de la colonne vertébrale peut alors être réalisée de façon à distinguer l'image du corps vertébral, seul concerné par l'ostéoporose, de celle de l'arc postérieur. Il est avantageux d'apparier la table 29 et le bâti 20 de façon à les positionner avec un faible jeu, comme on le représente figure 8. Pendant la durée de l'examen, la table est immobilisée par des vérins de blocage non représentés ici.

Le choix d'une batterie de capteurs autorise comme on l'a vu l'emploi d'un seul mécanisme de translation du chariot 22. La simultanéité des mesures implique en outre un temps d'examen sensiblement réduit si on utilise les sources radioactives de l'art antérieur. Mais on peut avantageusement utiliser des sources radioactives de fluence (nombre de photons gamma émis par unité de surface et de temps) plus faible. Le temps d'acquisition des mesures en chaque point doit alors être prolongé, ce qui limite les gains de temps d'examen que l'on pouvait espérer ; par contre, il est désormais possible d'employer des sources beaucoup moins coûteuses et plus durables que le gadolinium 153, en particulier l'américium 241 et le baryum 133 dont la demi-vie est beaucoup plus importante. Une combinaison faisant appel à un rayonnement X convenablement filtré produit par un tube à rayons X et une source radioactive peut aussi être utilisée.

Le rayonnement X peut venir en remplacement de l'élément de faible énergie, par exemple l'américium 241.

La simplicité d'un mécanisme qui permet de réaliser des examens selon deux incidences perpendiculaires, qui nécessite cependant l'emploi d'une série de capteurs dont on peut tirer parti par l'emploi d'une source radioactive plus intéressante, justifie donc l'intérêt porté à cette invention.

## Revendications

1. Appareil de détermination du contenu minéral osseux d'un patient par absorption partielle de rayonnements ionisants, comprenant un bloc (27) constitué d'une source de rayonnements ionisants (61) et un autre bloc (28) constitué d'un détecteur de rayonnement placé vis-à-vis de la source en position de travail et séparé de celle-ci d'une distance suffisante pour permettre l'introduction de la matière organique à examiner, caractérisé en ce qu'un des blocs (27 ou 28) est lié par une articulation (60) à un premier bras (23) lui-même articulé sur un chariot mobile (22), suivant une direction de translation (T), de l'appareil, tandis que l'autre bloc (28 ou 27) est solidaire d'un second bras (24) articulé lui aussi sur le chariot mobile (22), de façon à permettre deux observations successives du patient resté immobile selon deux directions d'irradiation perpendiculaires.

2. Appareil de détermination du contenu minéral osseux selon la revendication 1, caractérisé en ce qu'il comprend un moteur (30) relié par une courroie crantée (31) au chariot mobile (22), ainsi que des glissières (21) horizontales guidant la translation de celui-ci selon la direction (T).

3. Appareil de détermination du contenu minéral osseux suivant l'une quelconque des revendications 1 ou 2, caractérisé en ce que l'axe de l'articulation (25) du premier bras (23) est parallèle à la direction de déplacement (T) du chariot (22).

4. Appareil de détermination du contenu minéral osseux selon l'une quelconque des revendications 1 ou 2, caractérisé en ce que l'axe de l'articulation (26) du second bras (24) est perpendiculaire à la direction de déplacement (T) du chariot mobile (22) et fait un angle de 45° avec la verticale.

5. Appareil de détermination du contenu minéral osseux suivant l'une quelconque des revendications 1 à 4, caractérisé en ce qu'il comprend des moyens de blocage (42, 53, 78) des deux bras (23, 24) et du bloc (27) articulé sur le premier bras (23) définissant deux positions préférentielles d'irradiation telles que les directions respectives d'incidence du rayonnement sont perpendiculaires.

6. Appareil de détermination du contenu minéral osseux suivant la revendication 1, caractérisé en ce que le bloc (28) détecteur de rayonnement comprend une pluralité de capteurs (92 et 93) qui permettent la détection des rayonnements ionisants non absorbés.

7. Appareil de détermination du contenu minéral osseux suivant la revendication 6, caractérisé en ce que les capteurs (92 et 93) du bloc détecteur (28) sont disposés sur plusieurs rangées perpendiculaires à la direction de déplacement (T) du chariot mobile, et en ce que leur disposition suivant une projection orthogonale sur un plan perpendiculaire à cette direction (T) définit des intervalles égaux entre deux capteurs et inférieurs aux diamètres de ceux-ci.

8. Appareil de détermination du contenu minéral osseux suivant les revendications 1 et 6, caractérisé en ce que la source de rayonnements ionisants (61) est constituée d'un ou plusieurs éléments radioactifs de demi-vie supérieure à cinq ans.

9. Appareil du contenu minéral osseux suivant les revendications 1 et 6, caractérisé en ce que la source de rayonnements ionisants (61) est constituée d'un élément radioactif de demi-vie supérieure à cinq ans et d'une source de rayons X.

10. Appareil de détermination du contenu minéral osseux selon la revendication 8, caractérisé en ce que les éléments de la source radioactive (61) sont l'américium 241 et le baryum 133.

**11.** Appareil de détermination du contenu minéral osseux suivant la revendication 6, caractérisé en ce que la source de rayonnements ionisants est constituée de gadolinium 153.

**12.** Appareil de détermination du contenu minéral osseux suivant la revendication 6, caractérisé en ce que les capteurs (92′et 93′)du bloc détecteur comprennent des photomultiplicateurs à photocathodes multiples (93′) et en ce que les capteurs sont disposés sur une rangée unique perpendiculaire à la direction de déplacement (T) du chariot mobile.

**13.** Procédé de détermination du contenu minéral de la colonne vertébrale d'un patient à l'aide de l'appareil suivant les revendications 1 à 12, caractérisé en ce qu'il consiste à déplacer selon deux opérations de balayage le chariot mobile (22) le long du patient en décubitus dorsal sur une table d'examen (29), les blocs (27 et 28) étant disposés au-dessus et en-dessous de lui pour un balayage, à sa gauche et à sa droite pour l'autre balayage, la table (29) étant retirée entre ces deux opérations pour permettre la rotation des deux bras (23 et 24), et à corriger par des moyens numériques les intervalles de temps entre la prise d'informations concernant les différents points situés à la même hauteur du patient par les différents capteurs (92, 93) du bloc détecteur (28).

## Claims

**1.** Apparatus for determining the osseous mineral content of a patient by partial absorption of ionizing rays, comprising a block (27) constituted by an ionizing radiation source (61) and another block (28) constituted by a radiation detector positioned facing the source in the working position and separated therefrom by a distance adequate for permitting the introduction of the organic material to be examined, characterized in that one of the blocks (27 or 28) is linked by an articulation (60) to a first arm (23), itself articulated to a mobile carriage (22), in a translation direction (T) of the apparatus, whilst the other block (28 or 27) is integral with a second arm (24) articulated to the mobile carriage (22), so as to permit two successive observations of the stationary patient in two perpendicular irradiation directions.

**2.** Apparatus for determining the osseous mineral content according to claim 1, characterized in that it comprises a motor (30) connected by a notched belt (31) to the mobile carriage (22), as well as horizontal slides (21) guiding the translation thereof in the translation direction (T).

**3.** Apparatus for determining the osseous mineral content according to claims 1 or 2, characterized in that the articulation axis (25) of the first arm (23) is parallel to the displacement direction (T) of the carriage (22).

**4.** Apparatus for determining the osseous mineral content according to claims 1 or 2, characterized in that the articulation axis (26) of the second arm (24) is perpendicular to the displacement direction (T) of the mobile carriage (22) and forms an angle of 45° with the vertical.

**5.** Apparatus for determining the osseous mineral content according to any one of the claims 1 to 4, characterized in that it comprises locking means (42, 53, 78) for the two arms (23, 24) and the block (27) articulated to the first arm (23) defining two preferred irradiation directions such that the respective incidence directions of the radiation are perpendicular.

**6.** Apparatus for determining the osseous mineral content according to claim 1, characterized in that the radiation detector block (28) comprises a plurality of sensors (92 and 93) permitting the detection of non-absorbed ionizing rays.

**7.** Apparatus for determining the osseous mineral content according to claim 6, characterized in that the sensors (92, 93) of the detector block (28) are arranged in several rows perpendicular to the displacement direction (T) of the mobile carriage and in that their arrangement in accordance with an orthogonal projection on a plane perpendicular to said direction (T) defines equal gaps between two sensors and smaller than the diameters thereof.

**8.** Apparatus for determining the osseous mineral content according to claims 1 and 6, characterized in that the ionizing radiation source (61) is constituted by one or more radioactive elements having a half-life exceeding five years.

**9.** Apparatus for determining the osseous mineral content according to claims 1 and 6, characterized in that the ionizing radiation source (61) is constituted by a radioactive element having a half-life exceeding five years and an X-ray source.

10. Apparatus for determining the osseous mineral content according to claim 8, characterized in that the elements of the radioactive source (61) are americium 241 and barium 133.

11. Apparatus for determining the osseous mineral content according to claim 6, characterized in that the ionizing radiation source is constituted by gadolinium 153.

12. Apparatus for determining the osseous mineral content according to claim 6, characterized in that the sensors (92', 93') of the detector block incorporate multiple photocathode photomultipliers (93') and in that the sensors are arranged in a single row perpendicular to the displacement direction (T) of the mobile carriage.

13. Process for determining the osseous mineral content of the spinal column of a patient with the aid of an apparatus according to any one of the claims 1 to 12, characterized in that it consists of moving the mobile carriage (22) along the patient in the supine position on an examination table (29) in two scanning operations, the blocks (27, 28) being positioned above and below the patient for one scan and to his left and right for the other scan, the table (29) being retracted between these two operations to permit the rotation of the two arms (23, 24) and for correcting by digital means the time intervals between taking the information concerning the different points located at the same height of the patient by the different sensors (92, 93) of the detector block (28).

**Patentansprüche**

1. Gerät zur Bestimmung des Mineralgehalts von Knochen eines Patienten durch teilweise Absorption von ionisierender Strahlung, enthaltend einen Block (27), der von einer Quelle ionisierender Strahlung (61) und einen weiteren Block (28), der von einem Strahlungsdetektor gebildet ist und der Quelle in Arbeitsrichtung in einem Abstand gegenübersteht, der ausreichend für die Einführung der zu untersuchenden organischen Materie ist, **dadurch gekennzeichnet,** daß einer der Blöcke (27 oder 28), über ein Gelenk (60) mit einem ersten Arm (23) verbunden ist, der seinerseits an einem beweglichen Schlitten in einer Querrichtung (T) der Vorrichtung gelenkig angebracht ist, während der andere Block (28 oder 27) fest mit einem zweiten Arm (24) verbunden ist, der ebenfalls an dem beweglichen Schlitten (22) gelenkig angebracht ist derart, daß zwei aufeinanderfolgende Beobachtungen des unbeweglich gehaltenen Patienten in zwei zueinander senkrechten Bestrahlungsrichtungen möglich sind.

2. Gerät zur Bestimmung des Mineralgehalts von Knochen nach Anspruch 1, **dadurch gekennzeichnet,** daß es einen Motor (30) enthält, der über ein Zahnrad (31) mit dem beweglichen Schlitten (22) verbunden ist, sowie horizontale Gleitschienen (21), die den Schlitten quer in der Richtung (T) führen.

3. Gerät zur Bestimmung des Mineralgehalts von Knochen nach einem der Ansprüche 1 oder 2, **dadurch gekennzeichnet,** daß die Gelenkachse (25) des ersten Arms (23) parallel zur Verstellrichtung (T) des Schlittens (22) ist.

4. Gerät zur Ermittlung des Mineralgehalts von Knochen nach einem der Ansprüche 1 oder 2, **dadurch gekennzeichnet,** daß die Gelenkachse (26) des zweiten Arms (24) senkrecht zur Verstellrichtung (T) des beweglichen Schlittens (22) ist und einen Winkel von 45° mit der Vertikalen bildet.

5. Gerät zur Bestimmung des Mineralgehalts von Knochen nach einem der Ansprüche 1 bis 4, **dadurch gekennzeichnet,** daß es Blockierungseinrichtungen (42, 53, 78) für die zwei Arme (23, 24) und für den Block (27), der an dem ersten Arm (23) angelenkt ist, enthält, die zwei bevorzugte Bestrahlungspositionen, wie beispielsweise die zueinander senkrechten Bestrahlungsrichtungen, definieren.

6. Gerät zur Bestimmung des Mineralgehalts von Knochen nach Anspruch 1, **dadurch gekennzeichnet,** daß der Strahlungsdetektorblock (28) mehrere Aufnehmer (92 und 93) enthält, die die Ermittlung von ionisierender, nicht absorbierter Strahlung erlauben.

7. Gerät zur Bestimmung des Mineralgehalts von Knochen nach Anspruch 6, **dadurch gekennzeichnet,** daß die Aufnehmer (92 und 93) des Detektorblocks (28) in mehreren zur Verstellrichtung (T) des beweglichen Schlittens senkrechten Reihen angeordnet sind, und daß ihre Anordnung in einer orthogonalen Projektion auf eine Ebene, die senkrecht zu dieser Richtung (T) ist, gleiche Intervalle zwischen zwei Aufnehmern und kleiner als deren Durchmesser definiert.

8. Gerät zur Bestimmung des Mineralgehalts von Knochen nach den Ansprüchen 1 und 6, **da-**

**durch gekennzeichnet,** daß die ionisierende Strahlungsquelle (61) von einem oder mehreren radioaktiven Elementen einer Halbwertszeit von mehr als 5 Jahren gebildet ist.

9. Gerät zur Bestimmung des Mineralgehalts von Knochen nach den Ansprüchen 1 und 6, **dadurch gekennzeichnet,** daß die ionisierende Strahlungsquelle (21) von einem radioaktiven Element einer Halbwertszeit von mehr als 5 Jahren und einer Röntgenstrahlenquelle gebildet ist.

10. Gerät zur Bestimmung des Mineralgehalts von Knochen nach Anspruch 8, **dadurch gekennzeichnet,** daß die Elemente der radioaktiven Quelle (61) Americium 241 und Barium 133 sind.

11. Gerät zur Bestimmung des Mineralgehalts von Knochen nach Anspruch 6, **dadurch gekennzeichnet,** daß die ionisierende Strahlungsquelle von Gadolinium 153 gebildet ist.

12. Gerät zur Bestimmung des Mineralgehalts von Knochen nach Anspruch 6, **dadurch gekennzeichnet,** daß die Aufnehmer (92' und 93') des Detektorblocks Photovervielfacher mit mehreren Photokathoden (93') enthalten, und daß die Aufnehmer in einer einzigen Reihe angeordnet sind, die senkrecht zur Verstellrichtung (T) des beweglichen Schlittens ist.

13. Verfahren zur Bestimmung des Mineralgehalts der Wirbelsäule eines Patienten mit Hilfe des Geräts nach den Ansprüchen 1 bis 12, **dadurch gekennzeichnet,** daß es umfaßt: Das Verstellen des beweglichen Schlittens (22) in zwei Durchgängen längs des auf dem Rücken auf einem Untersuchungstisch (29) liegenden Patienten, wobei die Blöcke (27 und 28) über und unter diesem für einen Durchgang und zu seiner Linken und seiner Rechten für den nächsten Durchgang angeordnet werden, der Tisch (29) zwischen diesen zwei Vorgängen zurückgezogen wird, um die Drehung der zwei Arme (23 und 24) zu ermöglichen, und das Korrigieren durch digitale Einrichtungen der Zeitintervalle zwischen der Aufnahme von Informationen über verschiedene Punkte gleicher Höhe des Patienten durch die verschiedenen Aufnehmer (92, 93) des Detektorblocks (28).

FIG. 1

FIG. 10

FIG. 2

FIG. 11

EP 0 253 742 B1

FIG. 3

FIG. 4

# FIG. 5

# FIG. 6

EP 0 253 742 B1

FIG. 7

EP 0 253 742 B1

FIG. 8

FIG 9